# EUROPEAN PATENT APPLICATION

(11) **EP 0 573 032 A2**
(43) Date of publication of application: **08.12.1993**
(21) Application number: 93108926.2
(22) Date of filing: 03.06.1993
(51) Int. Cl.: C07C 69/76, C07C 67/08

(54) **Process for preparing dimethyl 2,6-naphtalenedicarboxylate**

(30) Priority: 05.06.1992 JP 170038/92
(71) Applicant: MITSUBISHI PETROCHEMICAL CO., LTD., Chiyoda-ku Tokyo 100 (JP)
(72) Inventor: Mori, Yoshiaki, Yokkaichi-shi, Mie (JP); Mine, Norioki, Yokkaichi-shi, Mie (JP)
(74) Representative: Hansen, Bernd, Dr. Dipl.-Chem.

(57) **Abstract**

In esterification of 2,6-naphthalenedicarboxylic acid with methyl alcohol to prepare dimethyl 2,6-naphthalenedicarboxylate, an improvement comprising using ferric sulfate as a catalyst is disclosed. The reaction proceeds under mild conditions at a high rate to produce the ester in a high yield.

## Description

### FIELD OF THE INVENTION

This invention relates to a process for preparing dimethyl 2,6-naphthalenedicarboxylate, and more particularly to an improvement in a process for preparing dimethyl 2,6-naphthalenedicarboxylate comprising reacting 2,6-naphthalenedicarboxylic acid and methyl alcohol.

Dimethyl 2,6-naphthalenedicarboxylate is useful as a raw material for resins such as poly(ethylene naphthalate).

### BACKGROUND OF THE INVENTION

Preparation of dimethyl 2,6-naphthalenedicarboxylate by reacting 2,6-naphthalenedicarboxylic acid and methyl alcohol is known.

While sulfuric acid is the most common catalyst for esterification of carboxylic acids, the strong corrosiveness of sulfuric acid requires reactors of expensive anticorrosive materials. Besides, dimethylether is produced as by-product. Therefore, sulfuric acid is not a suitable catalyst for industrial production.

In order to overcome the above-described problems of acid catalysts, it has been proposed to use a metallic catalyst for esterification between 2,6-naphthalenedicarboxylic acid and methyl alcohol. Metallic catalysts proposed includes molybdenum trioxide as disclosed in JP-A-50-83361 (the term "JP-A" as used herein means an "unexamined published Japanese patent application") and beryllium sulfate, bismuth sulfate, ceric sulfate, palladium sulfate, vanadyl sulfate, zirconium sulfate, and tetra-n-butyl titanate as disclosed in JP-A-51-8252.

However, verification experiments conducted by the present inventors revealed that the reaction in the presence of these metallic catalysts has a low rate of reaction, failing to attain a sufficient yield (see Comparative Examples 4 to 7 hereinafter described). In addition, these catalysts are generally expensive and are not industrially advantageous.

On the other hand, JP-A-51-8252 supra describes that iron oxide, iron chloride or iron powder, while effective in the esterification of terephthalic acid, exhibits substantially no effect in the esterification of 2,6-naphthalenedicarboxylic acid (see Comparative Examples 7 to 9 of Table 3 of the publication). A sulfate of cobalt or nickel, included under the same group of iron, is also low in effect as a catalyst for the esterification of 2,6-naphthalenedicarboxylic acid (see Comparative Examples 2 and 3 hereinafter described).

### SUMMARY OF THE INVENTION

An object of the present invention is to provide an improvement in the process for producing dimethyl 2,6-naphthalenedicarboxylate comprising esterifying 2,6-naphthalenedicarboxylic acid with methyl alcohol, the improvement achieving a high reaction rate and a high yield under mild conditions.

The present invention relates to a process for producing dimethyl 2,6-naphthalenedicarboxylate comprising reacting 2,6-naphthalenedicarboxylic acid and methyl alcohol in the presence of ferric sulfate [Fe₂(SO₄)₃].

### DETAILED DESCRIPTION OF THE INVENTION

2,6-Naphthalenedicarboxylic acid which can be used as a starting compound may be obtained through any of known processes, for example, (1) oxidation of a 2,6-dialkylnaphthalene, (2) thermal transition of a naphthalenedicarboxylic acid other than 2,6-naphthalenedicarboxylic acid or (3) disproportionation of naphthoic acid. 2,6-Naphthalenedicarboxylic acid containing a 2,6-naphthalenedicarboxylic acid monoester may also be used.

The amount of ferric sulfate to be used as the catalyst in the present invention is preferably from 0.05 to 10 parts by weight, more preferably from 1 to 2 parts by weight, per 100 parts by weight of the starting 2,6-naphthalenedicarboxylic acid. If the catalyst amount is less than 0.05% by weight, a sufficient catalysis could not be exerted. If it exceeds 10% by weight, no further improvement in catalysis will be obtained. Ferric sulfate may contain water of crystallization, but in this case the amount of the catalyst should be calculated as reduced to the anhydride.

The amount of methyl alcohol to be used for esterification is preferably from 2 to 20 parts by weight, more preferably from 3 to 10 parts by weight, per part by weight of 2,6-naphthalenedicarboxylic acid. If the methyl alcohol is less than the double the weight of 2,6-naphthalenedicarboxylic acid, a sufficient reaction rate could not be obtained. If it exceeds 20 times the weight, no further improvement in reaction rate or yield is obtained, only necessitating a larger reactor.

The esterification reaction is carried out at a temperature usually of from 130°C to 300°C, preferably from 150°C to 200°C. The reaction pressure depends mainly on the vapor pressure of methyl alcohol and water by-produced at the temperature used and mainly on the pressure of atmospherical inert gas. The reaction time is varied usually from about 10 minutes to about 5 hours, and preferably from 1 to 3 hours, by the catalyst amount and the reaction temperature. The reaction may be either in a batch system or in a continuous system.

After completion of the reaction, dimethyl 2,6-naphthalenedicarboxylate produced is collected by filtration, washed with methyl alcohol, and dried. If desired, the product may be subjected to distillation to obtain highly purified dimethyl 2,6-naphthalenedicarboxylate.

The present invention will now be illustrated in greater detail with reference to Examples in view of Comparative Examples, but it should be understood that the present invention is not construed as being limited thereto. All the parts are by weight unless otherwise indicated, and all the percents given for conversions (yields) are mol percents based on the starting material.

### EXAMPLES 1 AND 2

In a 200 mℓ stainless steel autoclave equipped with a stirrer were charged 100 parts (10 g) of 2,6-naphthalenedicarboxylic acid, 600 parts of methyl alcohol, and 1 part of ferric sulfate [Fe₂(SO₄)₃]. The mixture was heated to a prescribed temperature with stirring, kept at that temperature for a prescribed temperature to conduct esterification.

After completion of the reaction, the autoclave was cooled to room temperature, and the resulting slurry was filtered. The filter cake and the filtrate were each analyzed by high pressure liquid chromatography to determine 2,6-naphthalenedicarboxylic acid and also by gas chromatography to determine dimethyl 2,6-naphthalenedicarboxylate to obtain a conversion (yield). The results obtained are shown in Table 1 below.

**TABLE 1**

| Example No. | Reaction Temperature (°C) | Reaction Time (hr) | Yield (%) |
|---|---|---|---|
| 1 | 180 | 1.5 | 95 |
| 2 | 200 | 1.5 | 95 |

### COMPARATIVE EXAMPLE 1

Esterification was carried out in the same manner as in Example 2, except for using no ferric sulfate. The conversion of the reaction was found to be 1.5%.

### COMPARATIVE EXAMPLES 2 TO 7

In a 200 mℓ stainless steel autoclave equipped with a stirrer were charged 100 parts of 2,6-naphthalenedicarboxylic acid, 600 parts of methyl alcohol, and 1 part of each of the catalysts shown in Table 2 below. The mixture was heated to 200°C with stirring, kept at that temperature for 1.5 hours to conduct esterification.

After completion of the reaction, the reaction mixture was worked up in the same manner as in the foregoing Examples to obtain a conversion. The results obtained are shown in Table 2.

**TABLE 2**

| Compar. Example No. | Esterification Catalyst | Reaction Temperature (°C) | Reaction Time (hr) | Yield (%) |
|---|---|---|---|---|
| 1 | none | 200 | 1.5 | 1.5 |
| 2 | cobalt sulfate | 200 | 1.5 | 12 |
| 3 | nickel sulfate | 200 | 1.5 | 7.0 |
| 4 | molybdenum trioxide | 200 | 1.5 | 77 |
| 5 | bismuth sulfate | 200 | 1.5 | 18 |
| 6 | palladium sulfate | 200 | 1.5 | 24 |
| 7 | tetra-n-butyl titanate | 200 | 1.5 | 77 |

The present invention thus provides an industrially advantageous process for preparing dimethyl 2,6-naphthalenedicarboxylate, in which the catalyst used is cheap, and the reaction proceeds under mild conditions to attain a high reaction rate and a high yield.

While the invention has been described in detail and with reference to specific examples thereof, it will be apparent to one skilled in the art that various changes and modifications can be made therein without departing from the spirit and scope thereof.

## Claims

1. A process for producing dimethyl 2,6-naphthalenedicarboxylate comprising reacting 2,6-naphthalenedicarboxylic acid and methyl alcohol in the presence of ferric sulfate [Fe₂(SO₄)₃].

2. The process as claimed in claim 1, wherein ferric sulfate is used in an amount of 0.05 to 10 parts by weight per 100 parts by weight of 2,6-naphthalenedicarboxylic acid.

3. The process as claimed in claim 1, wherein methyl alcohol is used in an amount of 2 to 20 parts by weight per part by weight of 2,6-naphthalenedicarboxylic acid.

4. The process as claimed in claim 1, wherein the reaction is conducted at a temperature of from 130°C to 300°C.
